# EUROPÄISCHE PATENTSCHRIFT

(11) **EP 4 142 866 B1**
(45) Veröffentlichungstag und Bekanntmachung des Hinweises auf die Patenterteilung: **29.10.2025**
(21) Anmeldenummer: 21719865.4
(22) Anmeldetag: 09.04.2021
(51) Int. Cl.: A61N 1/375

(54) **KOPFTEIL EINER IMPLANTIERBAREN MEDIZINISCHEN VORRICHTUNG, VERFAHREN ZUR HERSTELLUNG DES KOPFTEILS SOWIE EINE IN DAS KOPFTEIL FÜGBARE STECKERANORDNUNG**
HEAD PART OF AN IMPLANTABLE MEDICAL DEVICE, METHOD FOR PRODUCING A HEAD PART, AND PLUG ASSEMBLY WHICH CAN BE FITTED INTO THE HEAD PART
PARTIE DE TÊTE D'UN DISPOSITIF MÉDICAL IMPLANTABLE, PROCÉDÉ DE PRODUCTION D'UNE PARTIE DE TÊTE ET ENSEMBLE FICHE QUI PEUT ÊTRE AJUSTÉ DANS LA PARTIE DE TÊTE

(30) Priorität: 28.04.2020 DE 102020205350
(43) Veröffentlichungstag der Anmeldung: 08.03.2023
(73) Patentinhaber: Neuroloop GmbH, 79110 Freiburg (DE)
(72) Erfinder: KIMMIG, Fabian, 79110 Freiburg (DE); BORETIUS, Tim, 79110 Freiburg (DE)
(74) Vertreter: Rösler, Uwe
(86) Internationale Anmeldenummer: PCT/EP2021/059291
(87) Internationale Veröffentlichungsnummer: WO 2021/219344

(56) Entgegenhaltungen:
- WO-A1-2018/222973
- WO-A1-2018/222973
- WO-A2-2007/059386
- US-A1- 2003 163 171
- US-A1- 2005 043 765
- US-A1- 2007 179 550
- US-A1- 2020 077 953
- US-B1- 10 471 251

## Beschreibung

### Technisches Gebiet

Die Erfindung bezieht sich auf ein Kopfteil einer implantierbaren medizinischen Vorrichtung, dessen Herstellverfahren sowie eine in das Kopfteil fügbare Steckeranordnung. Das Kopfteil weist ein Kopfteilgehäuse auf, das wenigstens eine sacklochartige Steckerkontaktbuchse mit einer Buchsenöffnung sowie einen der Buchsenöffnung axial gegenüberliegenden Buchsenboden besitzt, längs der in koaxialer Anordnung und axial serieller Abfolge wenigstens ein elektrisch leitendes Kontaktringelement sowie ein elektrisch isolierender, elastisch verformbarer Dichtungsring gefügt sind, die von einer erstarrten Vergussmasse umschlossen sind.

### Stand der Technik

Implantierbare medizinische Vorrichtungen zum Zwecke der elektrischen Stimulation lokaler intrakorporaler Gewebe- oder Nervenbereiche, kurz implantierbare Impulsgeber (IPG), beispielsweise für herztherapeutische Defribillations-, Schrittmacher- sowie Resynchronisationsanwendungen, für neurostimulationstherapeutische Maßnahmen, wie bspw. Rückenmarkstimulation, Hirnstimulation oder Vagusnervstimulation, um nur einige zu nennen, weisen in aller Regel ein in sich abgeschlossenes Gehäuse auf, in dem Komponenten zur elektrischen Pulsgeneration enthalten sind, so zumindest eine elektrische Energiequelle und eine mit dieser verbundenen, elektrischen Schaltungsstruktur. Zudem schließt an das Gehäuse ein sogenanntes Kopfteil an, in dem eine mit der Energiequelle bzw. der elektrischen Schaltungsstruktur verbundene, elektrische Kontaktanordnung enthalten ist, in die eine mit dem Kopfteil fluiddicht abschließende Stecker Anordnung fügbar ist, die mit elektrischen Zu- und Ableitungen zum Zwecke einer intrakorporalen lokalen Applikation elektrischer Stimulationssignale sowie gegebenenfalls der Zuführung intrakorporal lokal abgegriffener elektrischer Signale zur Gehäuseseitig vorhandenen elektrischen Schaltungsstruktur, kontaktiert ist.

In der Druckschrift EP 2 134 418 B1 ist ein gattungsgemäßes Kopfteil einer implantierbaren medizinischen Vorrichtung beschrieben, das zwei längs einer Fügenaht zusammenfügbare Kopfteilgehäusehälften umfasst, in die jeweils in serieller Abfolge durch Zwischenwände beabstandet, halbzylinderförmige Ausnehmungen eingebracht sind, in die in jeweils seriell abwechselnder Reihenfolge elektrisch leitende Kontaktringelemente sowie elektrisch isolierende Dichtungsringe eingesetzt sind. Das Kopfteil umfasst somit eine Anordnung koaxial zueinander ausgerichteter und elektrisch isolierter Kontaktringelemente, zu deren elektrischen Kontaktierung ein seitlicher Zugang im Kopfteil vorgesehen ist, durch den eine elektrische Steckeranordnung in einen von sämtlichen ringförmigen Kontaktringelementen umfassten Hohlraum fluiddicht fügbar ist.

Die Druckschrift DE 10 2012 010 901 A1 offenbart ein Verfahren zum Positionieren und Halten von elektrischen Kontakten und Dichtungen innerhalb eines Kopfteils zur elektrischen Kontaktierung an eine medizinische implantierbare Vorrichtung. In das aus einem bioverträglichen und elektrisch isolierenden Material bestehende Kopfteilgehäuse ist einseitig eine sacklochartige Bohrung eingebracht, in der in abwechselnder Reihenfolge elektrisch leitende Kontaktringe und ringförmige Dichtelemente eingebracht sind, die gemeinsam einen Hohlraum umfassen, in den eine stiftförmige Steckeranordnung fügbar ist. Jeder der einzelnen ringförmigen Kontaktringe ist innerhalb des Kopfteils über eine elektrische Verbindungsleitung mit innerhalb des Gehäuses der medizinische implantierbare Vorrichtung befindlichen elektrischen Komponenten verbunden.

In der Druckschrift DE 20 2013 012 073 U1 ist eine Steckerbohrung-Modulbaugruppe offenbart, zu deren Montage eine Anzahl von Kontaktringen und Dichtelementen in abwechselnder Reihenfolge längs eines stiftförmigen Montagewerkzeuges angeordnet ist. Mittels einer Spannvorrichtung werden sämtliche längs des Montagewerkzeuges aufsitzenden Kontaktringe und Dichtelemente unter Aufbringung einer axialen Fügekraft gegeneinander verspannt. Zum Zwecke der Konservierung der Fügekraft dient ein mittels einer Madenschraube axialfest auf dem Montagewerkzeug aufsitzendes Hülsenelement, das zusammen mit einem endseitigen Montagewerkzeugkopf die Anordnung aus Kontaktringen und Dichtelementen axial beidseitig begrenzt. In diesem verspannten Zustand erfolgt ein Vergießen der Anordnung mit einer aushärtbaren Vergussmasse, die im erstarrten Zustand die Fügekraft aufnimmt.

Anstelle einer Madenschraube als endseitige Fixierhilfe zur Konservierung der auf die längs des stiftförmigen Montagewerkzeuges angeordneten Reihenfolge aus Kontaktring- und Dichtringelementen wirkenden axialen Fügekraft ist in der Druckschrift WO 2019/115176 A1 vorgeschlagen, eine mit einer Öffnung und Innengewinde versehene Montageplatte einzusetzen, in deren Innengewindeöffnung das stiftförmige Montagewerkzeug mit einem an dessen Ende angeordnetes Außengewinde gefügt ist.

Die Druckschrift US2008/0077190 A1 offenbart ein Kopfteil zur elektrischen Kontaktierung eines medizinischen Implantats, in das eine Steckereinheit fluiddicht fügbar und elektrisch kontaktierbar ist. Zusätzlich zu den Stecker- und Kopfteilseitig vorgesehenen Kontaktelektroden ist in beiden Komponenten jeweils ein optischer Übertragungskanal vorgesehen, die bei Ineinanderfügung beider Komponenten zur Übertragung optischer Signale optisch aneinander koppeln.

Die Druckschrift WO 2018/222973 A1 offenbart eine implantierbare medizinische Vorrichtung, in Form eines IPG mit Kopfteil, in das wenigstens eine sacklochartige Ausnehmung als Steckerbuchse zur Einfügung eines elektrischen Kontaktsteckers eingebracht ist. Die sacklochartige Ausnehmung besitzt ein geschlossenes Lochende.

Die Druckschrift US 2003/0163171 A1 offenbart ein Kopfteil, das ausschließlich zur elektrischen Kontaktierung zwischen innerhalb des Kopfteils vorgesehenen elektrisch leitende Kontaktringen und den elektrisch leitenden Verbindungsabschnitten längs der Steckeranordnung dient. Das Kopfteil sieht eine Ausnehmung vor, in die eine elektrische Kontaktierungseinrichtung in Form einer seriellen Abfolge von Kontaktring- und Dichtringelementen einsetzbar ist. Nach Einfügen der Steckerkontaktanordnung in die sacklochartige Öffnung der Kontaktierungseinrichtung wird das Ende der Steckerkontaktanordnung mit Hilfe einer Fixierschraube seitlich fixiert, um ein unkontrolliertes Herausrutschen der Steckeranordnung aus dem Kopfteil zu vermeiden.

### Darstellung der Erfindung

Der Erfindung liegt die Aufgabe zugrunde ein Kopfteil einer implantierbaren medizinischen Vorrichtung, dessen Herstellverfahren sowie eine in das Kopfteil fügbare Steckeranordnung derart zu modifizieren, so dass es neben der bisher bekannten Erzeugung, Übertragung und Applikation von elektrischen Stimulationssignalen möglich sein soll den Funktionsumfang von implantierbaren Impulsgebern (IPG) zu vergrößern ohne dabei deren Baugröße nennenswert zu ändern.

Die Lösung der der Erfindung zugrunde liegenden Aufgabe ist im Anspruch 1 angegeben. Gegenstand des Anspruches 11 ist eine implantierbare Steckeranordnung, die passend zum Fügen in das Kopfteil ausgebildet ist. Im Anspruch 13 ist ein Verfahren zur Herstellung des Kopfteils angegeben. Den Erfindungsgedanken in vorteilhafter Weise weiterbildende Merkmale sind Gegenstand der Unteransprüche sowie der Beschreibung, insbesondere unter Bezugnahme auf die illustrierten Ausführungsbeispiele zu entnehmen.

Das lösungsgemäße Kopfteil einer implantierbaren medizinischen Vorrichtung, mit einem Kopfteilgehäuse, das wenigstens eine sacklochartige Steckerkontaktbuchse mit einer Buchsenöffnung sowie einem der Buchsenöffnung axial gegenüberliegenden Buchsenboden besitzt, längs der in koaxialer Anordnung und axial serieller Abfolge wenigstens ein elektrisch leitendes Kontaktringelement sowie ein elektrisch isolierender, elastisch verformbarer Dichtungsring gefügt sind, die von einer erstarrten Vergussmasse umschlossen sind, zeichnet sich dadurch aus, dass der Buchsenboden von einem von der erstarrten Vergussmasse zumindest teilweise umschlossenen Verbindungsmittel begrenzt ist, an das wenigstens ein in die sacklochartige Steckerkontaktbuchse offen mündender Medienkanal angebracht ist, der von der erstarrten Vergussmasse zumindest teilweise umschlossenen ist und ein dem Verbindungsmittel abgewandtes und mit einer Medienquelle verbundenes Kanalende aufweist. Der wenigstens eine Medienkanal ist in Form einer Fluidleitung ausgebildet. Vorzugsweise ist die Medienquelle mit einer Fluidpumpe verbunden, die vorzugsweise wiederrum mit einem Fluidreservoir verbunden ist. Alternativ ist der Medienkanal anstelle mit einer Medienquelle mit einer Mediensenke verbunden, längs der ein aktives Fördermittel mit Auffangbehälter oder lediglich ein für die Aufnahme eines Fluids vorgesehener Auffangbehälter angeordnet ist.

Die der Erfindung zugrunde liegende Idee macht sich die mit dem Innengewinde versehene Öffnung innerhalb der Montageplatte, die in der vorstehend erläuterten Druckschrift WO 2019/115176 A1 beschrieben ist und dort nach Entnahme des Montagewerkzeuges aus dem fertiggestellten Kopfteil ansonsten funktionslos bleibt, zunutze, um durch die Öffnung hindurch einen Medienstrom beliebiger Art zu richten.

Die im vorgenannten Stand der Technik als eine Art verlorenes Bauelement anzusehende Montageplatte, die vollständig von der Vergussmasse des Kopfteils umhüllt ist und innerhalb der erstarrten Vergussmasse für den weiteren Betrieb des Kopfteils sowie auch des mit dem Kopfteil verbundenen implantierbaren Impulsgeber (IPG) ansonsten funktionslos ist, bildet die zentrale Komponente des modifizierten, lösungsgemäßen Kopfteils, die neben der bereits angesprochenen Funktion als Koppelöffnung, durch die ein Medienstrom gerichtet werden kann, zudem als mechanische Halterung bzw. Stützstruktur für die endseitige Anbringung eines Medienkanals dient.

Der Medienkanal der in der einfachsten Ausführungsform als Hohlkanal ausgebildet ist, endet einseitig an der im Weiteren als Verbindungsmittel bezeichneten Montageplatte, deren ursprüngliche Funktion als Hilfskomponente zur Erzeugung einer axialen Fügekraft durch Vorsehen eines Innengewindes, wie eingangs erläutert, erhalten bleibt.

Der vom Verbindungsmittel abgehende Medienkanal ist von der Vergussmasse des Kopfteils umgeben und durchragt das Kopfteil in einer bevorzugten Ausführungsform nach außen in Form einer weiterführenden Hohlleitung, deren Hohlleitungsende mit einer Medienquelle verbunden ist. Der außerhalb des Kopfteils führende Medienkanal sowie auch die mit dem Medienkanal endseitig verbundene Medienquelle sind in einem ersten Ausführungsbeispiel separat sowohl vom Kopfteil als auch die unmittelbar daran anschließende implantierbare medizinische Vorrichtung, in der ein Pulsgenerator enthalten ist, angeordnet und ausgebildet. Alternativ sieht ein weiteres Ausführungsbeispiel die Integration des Medienkanals sowie der mit diesem verbundenen Medienquelle innerhalb der implantierbaren, medizinischen Vorrichtung vor.

Der in das bzw. aus dem Kopfteil führende Medienkanal sowie auch die mit dem Medienkanal einseitig verbundene Medienquelle ist nach Art des Mediums angepasst ausgebildet.

Der Medienkanal ist als Fluidkanal ausgebildet, d.h. das Medium ist gasförmig oder flüssig und wird über den Medienkanal fluiddicht aus der als Fluidreservoir ausgebildeten Medienquelle, vorzugsweise unterstützt durch eine Fluidfördereinheit, die mit dem Fluidreservoir in Form einer Baueinheit kombiniert oder längs des Medienkanals angeordnet ist, in Richtung der sacklochartigen Steckerkontaktbuchse, in die der Medienkanal offen mündend, gefördert.

In Kombination mit der vorstehenden Ausbildung dient der Medienkanal, längs dem ein lichtleitendes Fluid strömt, vorzugsweise zusätzlich als Lichtleiter. Als weitere Medienquelle dient in diesem Fall eine Lichtquelle, die Licht emittiert, das über den längs des Medienkanals verlaufenden Lichtleiter an das offen in die sacklochartige Steckerkontaktbuchse mündende Lichtleiterende geleitet wird.

Für eine mechanisch robuste Lagerung des endseitig mit dem Verbindungsmittel verbundenen Medienkanals sieht das Verbindungsmittel eine den in die sacklochartige Steckerkontaktbuchse offen mündenden Medienkanal umfassende Fügekontur vor, die derart ausgestaltet und angeordnet ist, dass ein innerhalb der Steckerkontaktbuchse angeordneter zweiter Medienkanal an den in die sacklochartige Steckerkontaktbuchse offen mündenden Medienkanal ankoppelbar ist. Der zweite innerhalb der Steckerkontaktbuchse angeordnete Medienkanal ist Teil einer lösungsgemäß ausgebildeten implantierbaren Steckeranordnung, die zum Fügen in die sacklochartige Steckerkontaktbuchse des vorstehend bezeichneten Kopfteils entsprechend ausgebildet und angepasst ist. Insbesondere verfügt die Steckeranordnung über einen Steckerkorpus mit wenigstens einem innenliegenden Hohlkanal, der dem vorstehend bezeichneten zweiten Medienkanal entspricht und einseitig am distalen Steckerkorpusende mündet. Überdies weist das distale Steckerkorpusende eine Fügekontur auf, die im gefügten Zustand der Steckeranordnung innerhalb der Steckerkontaktbuchse einen bündigen Übergang des Hohlkanals zu dem in die sacklochartige Steckerkontaktbuchse offen mündenden Medienkanal gewährleistet. Hierdurch soll eine möglichst verlustfreie Ankopplung und Weiterleitung bzw. Übertragung des Mediums vom ersten Medienkanal in den als zweiten Medienkanal dienenden Hohlkanal realisierbar sein.

Die implantierbare Steckeranordnung ist ansonsten in an sich bekannter Weise zur Übertragung elektrischer Signale ausgebildet und weist in Steckerlängserstreckung eine serielle Abfolge von Steckerkontaktringen sowie Dichtungsringe bzw. elektrische Isolationsringe auf, entsprechend der Anzahl und Anordnung der elektrischen Kontaktringelemente und Dichtungsringen längs der Steckerkontaktbuchse.

Im gefügten Zustand der implantierbaren Steckeranordnung innerhalb der kopfteilseitig vorgesehenen Steckerkontaktbuchse bleibt die elektrische Funktionalität der implantierbaren Vorrichtung zur Applikation elektrischer Stimulationssignale trotz modifiziertem Kopfteil und modifizierter Steckeranordnung uneingeschränkt erhalten. Über den zusätzlich vorgesehenen kopfteilseitigen sowie auch steckerseitigen Medienkanal, der von der Steckeranordnung gemeinsam mit der abführenden elektrischen Leitung oder separiert zu dieser abführt, lässt sich ein zusätzlicher intrakorporal lokal applizierbarer Medienaustrag, beispielsweise in Form eines Gas- oder Flüssigkeitsstroms, gegebenenfalls in Kombination mit einer zusätzlichen Lichtübertragung realisieren.

In einer weiteren bevorzugten Ausführungsform ist innerhalb des Kopfteils längs des von der erstarrten Vergussmasse zumindest teilweise umschlossenen Medienkanals wenigstens ein Sensor integriert, der das jeweils innerhalb des Medienkanals geführte Medium zumindest quantitativ in Bezug auf Fördervolumen, Förderrate oder Lichtintensität etc., zu detektieren bzw. sensorisch zu erfassen vermag. Je nach Art und Ausführung des wenigstens einen Sensors ist dessen Anordnung innerhalb des Kopfteils längs des Medienkanals geeignet vorzunehmen, beispielsweise ist es bietet es sich an, den wenigstens einen Sensor mit dem wenigstens einen innerhalb des Kopfteils angebrachten elektrisch leitenden Kontaktringelement elektrisch und/oder mechanisch direkt oder indirekt zu koppeln. Als mögliche Sensoren kommen Sensoren aus der nachfolgenden Gruppe infrage: Ultraschallsensor, optischer Sensor, Drucksensor, Hallsensor, Flusssensor, Nadelsensoren, Fluoreszenzsensoren, Drucksensoren etc..

Der wenigstens eine längs des Medienkanals angeordnete Sensor ist vorzugsweise mit einer elektrischen Energiequelle verbunden, die zur elektrischen Energieversorgung des Pulsgenerators innerhalb der medizinischen Vorrichtung dient. Zudem ist der wenigstens eine Sensor mit einer Auswerte- und Steuereinheit verbunden, die mit einer zusätzlichen Speichereinheit und/oder mit der Medienquelle verbunden ist. Auf diese Weise ist es möglich, den Istzustand des längs des Medienkanals strömenden oder geleiteten Mediums zu erfassen und die Medienquelle in Abhängigkeit des Istzustandes anzusteuern bzw. zu regeln.

Das lösungsgemäße konfektionierte Kopfteil sowie die funktionell modifizierte Steckeranordnung zur zusätzlichen Übertragung eines Mediums in Form eines Fluids, vorzugsweise und eines Lichtstroms, belässt die Form und Größe bekannter gattungsgemäßer medizinischer Implantate weitgehend unverändert, erweitert jedoch signifikant die Funktionalität und die damit verbundene therapeutische Wirkweise vergleichbarer Implantate. So ist es möglich, neben der elektrischen Stimulation intrakorporaler lokaler Gewebe- und oder Nervenbereiche zusätzlich ein Fluid und gegebenenfalls Licht zu applizieren.

Durch die kombinierte Nutzung des originär als Montageplatte dienenden Verbindungsmittels als Tragstruktur sowie als Verbindungsflansch für zwei auf Stoß aneinander angrenzende Medienkanäle, eröffnet sich eine neuartige Herstellungsweise für ein derartiges Kopfteil. So dient das Verbindungsmittel in einem ersten Verfahrensschritt als Trägerplatte im herkömmlichen Sinn, d.h. das Verbindungsmittel wird zusammen mit dem wenigstens einen Kontaktringelement sowie dem wenigstens einen Dichtungsring längs eines stabförmigen Montagewerkzeuges angeordnet und längs des stabförmigen Montagewerkzeuges durch Erzeugen einer axialen Spannkraft gegeneinander kraftschlüssig gefügt.

In einem nächsten Schritt wird der Medienkanal in Form einer Hohlleitung oder Hohlkanüle endseitig mit dem Verbindungsmittel derart gefügt, so dass der Medienkanal mittel- oder unmittelbar an der Öffnung innerhalb des Verbindungsmittels angrenzt. Beispielsweise kann der Medienkanal passgenau auf das rückseitig am Verbindungsmittel überstehende Ende des stabförmigen Montagewerkzeuges umhüllend gefügt werden.

Im Anschluss daran werden zumindest das längs des stabförmigen Montagewerkzeuges angeordnete und kraftschlüssig gefügte Verbindungsmittel, das wenigstens eine Kontaktringelement sowie der wenigstens eine Dichtungsring sowie der wenigstens eine mittel- oder unmittelbar an dem Verbindungsmittel angebrachte Medienkanal zumindest bereichsweise mit einer erstarrungsfähigen, in fließfähiger Form vorliegenden Vergussmasse ummantelt. Nachfolgend wird das stabförmige Montagewerkzeug von dem Verbindungsmittel, dem wenigstens einen Kontaktringelement sowie dem wenigstens einen Dichtungsring nach Erstarren der Vergussmasse, die in Form einer zumindest einen Teil des Kopfteilgehäuses bildenden, formstabilen Matrix die axiale Fügekraft mechanisch abfängt, gelöst und entfernt. Durch Entfernen des Montagewerkzeuges mündet der mittel- oder unmittelbar am Verbindungsmittel angebrachte Medienkanal einseitig offen in die sich durch Entfernen des Montagewerkzeuges ausbildende sacklochartige Steckerkontaktbuchse des Kopfteils.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:

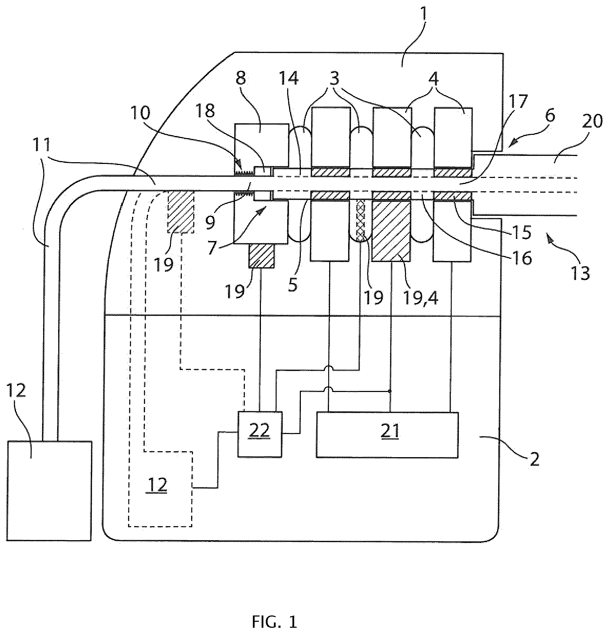

- Fig. 1: Darstellung eines mit einem zusätzlichen Medienkanal kombinierten Kopfteils eines implantierbaren Pulsgenerators mit einer entsprechend konfektionierten Steckeranordnung.

### Wege zur Ausführung der Erfindung, gewerbliche Verwendbarkeit

Fig. 1 zeigt eine schematisierte Darstellung eines Kopfteils 1, das mit einer als Pulsgenerator ausgebildeten implantierbaren, medizinischen Vorrichtung 2 zu einer Baueinheit kombiniert ist. In dem aus einer erstarrungsfähigen Vergussmasse gefertigten Kopfteil 1 ist in axial serieller Abfolge eine Vielzahl von elektrisch isolierenden, elastisch verformbaren Dichtungsringen 3 sowie elektrisch leitenden Kontaktringelementen 4 in jeweils abwechselnder Reihenfolge angeordnet. Die Dichtungsringe 3 sowie die elektrischen Kontaktringelemente 4 umfassen eine sacklochartige Steckerkontaktbuchse 5, die eine an der Außenwand des Kopfteils 1 zugängliche Buchsenöffnung 6 besitzt. Der der Buchsenöffnung 6 gegenüber liegende Buchsenboden 7 ist von einem Verbindungsmittel 8 begrenzt, das für die Fertigung des Kopfteils 1 und insbesondere für eine Fügekraft beaufschlagte, axiale Aneinanderreihung der Vielzahl an Dichtungsringen 3 sowie elektrischen Kontaktringelementen 4 als mechanisches Gegenlager für ein zu Montagezwecken benutztes Montagewerkzeug dient. Hierzu sieht das Verbindungsmittel 8 eine Öffnung 9 vor, längs der zumindest bereichsweise ein Innengewinde 10 eingebracht ist.

An bzw. in das Verbindungsmittel 8 im Bereich der Öffnung 9 ist ein Medienkanal 11 gefügt, der offen in die sacklochartige Steckerkontaktbuchse 5 mündet. Der Medienkanal 11 verläuft bereichsweise innerhalb des Kopfteils 1 und ist somit von der erstarrten Vergussmasse des Kopfteils 1 umgeben. Der als Hohlkanal ausgebildete Medienkanal 11 ragt seitlich aus dem Kopfteil 1 heraus und ist endseitig mit einer Medienquelle 12 verbunden. Die Medienquelle 12 stellt beispielsweise ein Medienreservoir dar, aus dem Medium, beispielsweise Gas oder eine Flüssigkeit vermittels einer Fördereinheit in kontrollierter Weise längs des Medienkanals 11 gefördert wird. In diesem Fall ist der Medienkanal 11 als Hohlkanal ausgebildet, durch den möglichst verlustfrei das gasförmige oder flüssige Medium in Richtung des Kopfteils 1 gefördert wird.

Die Medienquelle 12 kann zusätzlich auch als Lichtquelle ausgebildet sein. In diesem Fall gilt es ein möglichst lichttransparentes Fluid durch den Medienkanal 11 zu fördern, das zusätzlich eine Art Lichtleiter darstellt, längs dem das seitens der kombinierten Medienquelle 12 aus Fluid-und Lichtquelle Fluid und Licht in Richtung des Kopfteils 1 weitgehend verlustfrei geleitet wird.

In der sacklochartigen Steckerkontaktbuchse 5 ist zudem eine lösungsgemäß konfektionierte Steckeranordnung 13 gefügt, die einen an die Dimensionen und Formgebung der Steckerkontaktbuchse 5 angepassten Steckerkorpus 14 aufweist, an dessen Außenumfang entsprechend der Anordnung der elektrischen Kontaktringelemente 4 entsprechende Gegenkontaktelemente 15 angeordnet sind. Zwischen den Gegenkontaktelementen 15 sind elektrisch isolierende Steckerkorpusbereiche 16 vorgesehen. Betreffend die elektrische Kontaktierung der Steckerkontaktanordnung 13 innerhalb der sacklochartig ausgebildeten Steckerkontaktbuchse 5 des Kopfteils 1 unterscheidet sich die in Fig.1 dargestellte Vorrichtung nicht von vergleichbaren, gattungsgemäßen Steckeranordnungen.

In lösungsgemäßer Weise sieht die Steckerkontaktanordnung 13 einen den Steckerkorpus 14 mittig durchsetzenden zweiten Medienkanal 17 vor, der bündig im Bereich des Buchsenbodens 7 an den offen in die sacklochartige Steckerkontaktbuchse 5 mündenden Medienkanal 11 in Form einer Stoßverbindung angrenzt. Je nach Art und Ausbildung des Medienkanals 11, 17 gilt es, die Ankopplung zwischen dem kopfteilseitigen Medienkanal 11 und dem steckerseitigen zweiten Medienkanal 17 für eine verlustarme bzw. verlustfreie Ankopplung und Übertragung des jeweiligen Mediums entsprechend vorzunehmen. Optional ist hierzu innerhalb des Verbindungsmittels 8 eine zusätzliche Dichtungs- und/oder Fügekontur 18 eingebracht, die für einen nahtlosen Übergang zwischen dem Medienkanal 11 und 17 sorgt.

Optional ist längs des Medienkanals 11 und/oder 17 ein Sensor 19 innerhalb des Kopfteils 1 zur Detektion bzw. Überwachung des längs des Medienkanals 11, 17 geleiteten Mediums angeordnet. Je nach Art und Ausbildung des Sensors 19 ist dieser unmittelbar längs des Medienkanals 11, am Verbindungsmittel 8, an wenigstens einem der Dichtungsringe 3 oder an wenigstens einem der elektrischen Kontaktringelemente 4 angeordnet oder in diesen integriert. Die Art und Anbringung des wenigstens einen Sensors 19 richtet sich nach dem längs des Medienkanals 11, 17 übertragenen Mediums, beispielsweise Gas, Flüssigkeit und gegebenenfalls Licht. Als Sensor 19 kann in geeigneter Weise aus den nachfolgenden Sensortypen gewählt werden: Hallsensor, Optiksensor, Flusssensor, Ultraschallsensor, Temperatursensor, Drucksensor etc..

Die Steckerkontaktanordnung 13 ist mit einer abführenden Leitung 20 verbunden, die neben der wenigstens einen elektrischen Leitung den abführenden Medienkanal 17 umfasst. Über den abführenden Medienkanal 17 lässt sich an ausgewählten intrakorporalen Bereichen das Medium kontrolliert, d.h. dosiert zu therapeutischen Zwecken applizieren.

Das in Fig. 1 illustrierte Ausführungsbeispiel zeigt einen aus dem Kopfteil 1 führenden Medienkanal 11, der getrennt zum IPG 2 mit einer Medienquelle 12 verbunden ist. Gleichsam ist es möglich, den Medienkanal 11 innerhalb des Kopfteils 1 sowie des IPG's 2 zu führen und mit einer innerhalb des IPG's angeordneten Medienquelle zu verbinden, wie dies aus der strichlierten Darstellung in Fig. 1 zu entnehmen ist.

Innerhalb der als Pulsgenerator ausgebildeten implantierbaren, medizinischen Vorrichtung 2 ist neben den zur Pulserzeugung erforderlichen Komponenten 22 zusätzlich eine mit dem wenigstens einen Sensor 19 verbundene Auswerte- und Steuereinheit 22 angeordnet, die in Abhängigkeit des wenigstens einen Sensorsignals ein Steuer- oder Regelsignal zur Beeinflussung der Medienquelle 12 generiert. Auf diese Weise kann Einfluss auf den Fluss bzw. die Intensität des durch den Medienkanal 11, 17 geleiteten Mediums genommen werden.

### Bezugszeichenliste

- 1: Kopfteil
- 2: als Pulsgenerator ausgebildete implantierbare medizinische Vorrichtung, IPG
- 3: Dichtungsring
- 4: elektrisches Kontaktringelement
- 5: sacklochartige Steckerkontaktbuchse
- 6: Buchsenöffnung
- 7: Buchsenboden
- 8: Verbindungsmittel
- 9: Öffnung
- 10: Innengewinde
- 11: Medienkanal
- 12: Medienquelle
- 13: Steckerkontaktanordnung
- 14: Steckerkorpus
- 15: Gegenkontaktelement
- 16: isolierender Zwischenbereich
- 17: zweiter Medienkanal
- 18: Dichtungs-Fügekontur
- 19: Sensor
- 20: abführende Leitung
- 21: elektrische Komponenten des IPGs
- 22: Auswerte- und Steuereinheit

## Patentansprüche

1. Kopfteil (1) einer implantierbaren medizinischen Vorrichtung (2), mit einem Kopfteilgehäuse, das wenigstens eine sacklochartige Steckerkontaktbuchse (5) mit einer Buchsenöffnung (6) sowie einem der Buchsenöffnung (6) axial gegenüberliegenden Buchsenboden (7) besitzt, längs der in koaxialer Anordnung und axial serieller Abfolge wenigstens ein elektrisch leitendes Kontaktringelement (4) sowie ein elektrisch isolierender, elastisch verformbarer Dichtungsring (3) gefügt sind, die von einer erstarrten Vergussmasse umschlossen sind,
**dadurch gekennzeichnet, dass** der Buchsenboden (7) von einem von der erstarrten Vergussmasse zumindest teilweise umschlossenen Verbindungsmittel (8) begrenzt ist, an das wenigstens ein in die sacklochartige Steckerkontaktbuchse (5) offen mündender Medienkanal (11) angebracht ist, der von der erstarrten Vergussmasse zumindest teilweise umschlossenen ist und ein dem Verbindungsmittel (8) abgewandtes und mit einer Medienquelle (12) verbundenes Kanalende aufweist, und dass der wenigstens eine Medienkanal (11) in Form einer Fluidleitung ausgebildet ist.

2. Kopfteil nach Anspruch 1,
**dadurch gekennzeichnet, dass** die Medienquelle (12) eine mit einem Fluidreservoir verbundene Fluidpumpe ist oder
dass die Medienquelle (12) als Mediensenke ausgebildet ist.

3. Kopfteil nach Anspruch 1 oder 2,
**dadurch gekennzeichnet, dass** der Medienkanal (11) vom Verbindungsmittel (8) abgehend das Kopfteilgehäuse nach Außen durchragt und außerhalb des Kopfteils (1) mit der Medienquelle (12) verbunden ist.

4. Kopfteil nach einem der Ansprüche 1 bis 3,
**dadurch gekennzeichnet, dass** längs des von der erstarrten Vergussmasse zumindest teilweise umschlossenen Medienkanals (11) wenigstens ein Sensor (19) angebracht ist zur sensorischen Erfassung eines innerhalb des Medienkanals (11) vorhandenen Mediums.

5. Kopfteil nach einem der Ansprüche 1 bis 4,
**dadurch gekennzeichnet, dass** wenigstens ein Sensor (19) mit dem wenigstens einen elektrisch leitenden Kontaktringelement (4) elektrisch und/oder mechanisch verbunden ist.

6. Kopfteil nach Anspruch 4 oder 5,
**dadurch gekennzeichnet, dass** der wenigstens eine Sensor (19) aus der Gruppe der nachfolgenden Sensoren auswählbar ist: Ultraschallsensor, optischer Sensor, Drucksensor, Hall-Sensor, Flusssensor.

7. Kopfteil nach einem der Ansprüche 4 bis 6,
**dadurch gekennzeichnet, dass** der wenigstens eine Sensor (19) mit einer Auswerte- und Steuereinheit (22) verbunden ist, die mit einer Speichereinheit und/oder mit der Medienquelle (12) verbunden ist.

8. Kopfteil nach einem der Ansprüche 1 bis 7,
**dadurch gekennzeichnet, dass** das Verbindungsmittel (8) eine den in die sacklochartige Steckerkontaktbuchse (5) offen mündenden Medienkanal (11) umfassende Fügekontur (18) aufweist, die derart ausgestaltet und angeordnet ist, so dass ein innerhalb der Steckerkontaktbuchse (5) angeordneter zweiter Medienkanal (17) an den in die sacklochartige Steckerkontaktbuchse (5) offen mündenden Medienkanal (11) ankoppelbar ist.

9. Kopfteil nach einem der Ansprüche 1 bis 8,
**dadurch gekennzeichnet, dass** das Kopfteilgehäuse mechanisch und elektrisch mit einer als Pulsgenerator ausgebildeten implantierbaren, medizinischen Vorrichtung (2) verbunden ist.

10. Kopfteil nach Anspruch 9,
**dadurch gekennzeichnet, dass** die Medienquelle (12) in der implantierbaren, medizinische Vorrichtung (2) integriert ist.

11. Implantierbare Steckeranordnung ausgebildet und angepasst zum Einfügen in die sacklochartige Steckerkontaktbuchse (5) innerhalb des Kopfteils (1) nach einem der Ansprüche 1 bis 10,
**dadurch gekennzeichnet, dass** die Steckeranordnung (13) einen Steckerkorpus (14) mit wenigstens einem innenliegenden Hohlkanal aufweist, der einseitig am distalen Steckerkorpusende mündet, und
dass das distale Steckerkorpusende eine Fügekontur (18) aufweist, die im gefügten Zustand der Steckeranordnung (13) innerhalb der Steckerkontaktbuchse (5), eine bündige Fügung des Hohlkanals an den in die sacklochartige Steckerkontaktbuchse (5) offen mündenden Medienkanal (11) gewährleistet.

12. Steckeranordnung nach Anspruch 11,
**dadurch gekennzeichnet, dass** der Hohlkanal einen Medienkanal (11) nach Art des in die sacklochartige Steckerkontaktbuchse (5) offen mündenden Medienkanals (11) umfasst.

13. Verfahren zur Herstellung eines Kopfteils (1) nach einem der Ansprüche 1 bis 10,
**dadurch gekennzeichnet, dass** das Verbindungsmittel (8), das wenigstens eine Kontaktringelement (4) sowie der wenigstens eine Dichtungsring (3) längs eines stabförmigen Montagewerkzeuges angeordnet und längs des stabförmigen Montagewerkzeuges durch Erzeugen einer Fügekraft gegeneinander kraftschlüssig gefügt werden,
dass zumindest das längs des stabförmigen Montagewerkzeuges angeordnete und kraftschlüssig gefügte Verbindungsmittel (8), das wenigstens eine Kontaktringelement (4) sowie der wenigstens eine Dichtungsring (3) sowie der wenigstens eine mittel- oder unmittelbar an dem Verbindungsmittel (8) angebrachte Medienkanal (11) zumindest bereichsweise mit einer erstarrungsfähigen, in fließfähiger Form vorliegenden Vergussmasse ummantelt werden,
dass das stabförmige Montagewerkzeug von dem Verbindungsmittel (8), dem wenigstens einen Kontaktringelement (4) sowie dem wenigstens einen Dichtungsring (3) nach Erstarren der Vergussmasse, die in Form einer zumindest einen Teil des Kopfteilgehäuses bildenden, formstabilen Matrix die axiale Fügekraft mechanisch abstützt, gelöst und entfernt wird.

## Claims

1. Head part (1) of an implantable medical device (2), with a head part housing that has at least one blind hole-like plug contact socket (5) with a socket opening (6) and axially opposite the socket opening (6) a socket base (7), along which in coaxially arrangement and axially serial sequence, at last one electrically conducting contact ring element (4) as well as an electrically insulating, elastically deformable sealing ring (3) are attached and are surrounded by a solidified casting compound,
**characterised in that** the socket base (7) is delimited by a connection means (8), at least partially surrounded by the solidified casting compound (8), on which at least one media channel (11) opening into the blind hole-like plug contact socket (5) is attached, which is at least partially surrounded by the solidified casting compound and comprises a channel end facing away from the connection means (8) and connected to a media source (12), and **in that** the at least one media channel (11) is designed in the form of a fluid line.

2. Head part according to claim 1,
**characterised in that** the media source (12) is a fluid pump connected to a fluid reservoir or **in that** the media source (12) is designed as media sink.

3. Head part according to claim 1 or 2,
**characterised in that** starting from the connection means (8), the media channel (11) protrudes outwards through the head part housing and outside of the head part (1) is connected to the media source (12).

4. Head part according to any one of claims 1 to 3, **characterised in that** along the media channel (11) which is at least partially surrounded by the solidified casting compound, at least one sensor (19) is attached for the sensory recording of a medium present within the media channel (11).

5. Head part according to any one of claims 1 to 4, **characterised in that** at least one sensor (19) is electrically and/or mechanically connected to that at least one electrically conducting contact ring element (4).

6. Head part according to claim 4 or 5,
**characterised in that** the at least one sensor (19) is selectable from the group of the following sensors: ultrasound sensor, optical sensor, pressure sensor, Hall sensor, flow sensor.

7. Head part according to any one of claims 4 to 6, **characterised in that** the at least one sensor (19) is connected to an evaluation and control unit (22), which is connected to a storage unit and/or to the media source (12).

8. Head part according to any one of claims 1 to 7, **characterised in that** the connection means (8) comprises a joining contour (18) which encompasses the media channel (11) opening into the blind hole-like plug contact socket (5) and is designed and arranged so that a second media channel (17) arranged within the plug contact socket (5) can be connected to the medial channel (11) opening into the blind hole-like plug contact socket (5).

9. Head part according to any one of claims 1 to 8, **characterised in that** the head part housing is mechanically and electrically connected to an implantable medical device (2) designed as a pulse generator.

10. Head part according to claim 9,
**characterised in that** the media source (12) is integrated into the implantable medical device (2).

11. Implantable plug arrangement designed and adapted for insertion into the blind hole-like plug contact socket (5) within the head part (1) according to any one of claims 1 to 10,
**characterised in that** the plug arrangement (13) comprises a plug body (14) with at least one hollow channel present within it which at one end opens at the distal plug body end and
**in that** that distal plug body end comprises a joining contour (18) that in the joined state of the plug arrangement (13) within the plug contact socket (5), ensures flush joining of the hollow channel to the medial channel (11) opening into the blind hole-like plug contact socket (5).

12. Plug arrangement according to claim 11,
**characterised in that** the hollow channel comprises a media channel (11) in the style of the media channel (11) opening into the blind hole-like plug contact socket (5).

13. Method of manufacturing a head part (1) according to any one of claims 1 to 10,
**characterised in that** the connection means (8), the at least one contact ring element (4) as well as the at least one sealing ring (3) are arranged along a rod-like assembly tool and along the rod-like assembly tool are joined to each other in a force-fitting manner through the generation of a joining force,
**in that** at least the connection means (8) arranged and force-fittingly joined along the rod-like assembly tool, the at least one contact ring element (4) as well as the at least one sealing ring (3) as well as the one media channel (11) directly or directly attached to the connection means (8), are at least in parts surrounded with a solidifiable casting compound present in flowable form,
**in that** rod-like assembly tool is released and removed from the connection means (8), the at least one contact ring element (4) as well as the at least one sealing ring (3) after solidification of the casting compound, which in the form a dimensionally stable matrix forming at least one part of the head part housing, mechanically supports the axial joining force.

## Revendications

1. Partie de tête (1) d'un dispositif médical implantable (2), avec un boîtier de partie tête, qui possède au moins une prise de contact à fiche de type à trou borgne (5) avec une ouverture de prise (6) ainsi qu'un fond de prise (7) axialement opposé à l'ouverture de prise (6), le long de laquelle sont assemblés en disposition coaxiale et dans une suite en série au moins un élément annulaire de contact (4) électroconducteur, ainsi qu'une bague d'étanchéité (3) électriquement isolée, élastiquement déformable, qui sont entourés d'une masse de remplissage solidifiée,
**caractérisée en ce que** le fond de prise (7) est limité par un moyen de liaison (8) entouré au moins en partie par la masse de remplissage solidifiée sur lequel est placé au moins un conduit de milieu (11) débouchant ouvert dans la prise de contact à fiche de type à trou borgne (5), qui est entouré au moins en partie par la masse de remplissage solidifiée et comporte une extrémité de conduit éloignée du moyen de liaison (8) et reliée à une source de milieu (12) et **en ce qu'**au moins un conduit de milieu (11) est constitué sous la forme d'un conduit de fluide.

2. Partie de tête selon la revendication 1,
**caractérisée en ce que** la source de milieu (12) est une pompe de fluide reliée à un réservoir de fluide ou
***en ce que*** la source de milieu (12) est constituée sous la forme d'un puits de milieu.

3. Partie de tête selon la revendication 1 ou 2, **caractérisée en ce que** le conduit de milieu (11) traverse vers l'extérieur le boîtier de partie de tête en sortant du moyen de liaison (8) et est relié à la source de milieu (12) en dehors de la partie de tête (1)

4. Partie de tête selon l'une quelconque des revendications 1 à 3,
**caractérisée en ce qu'**au moins un capteur (19) est placé le long du conduit de milieu (11) entouré au moins en partie par la masse de remplissage solidifiée pour la saisie de détection d'un milieu présent à l'intérieur du conduit de milieu (11).

5. Partie de tête selon l'une quelconque des revendications 1 à 4,
**caractérisée en ce qu'**au moins un capteur (19) est relié électriquement et/ou mécaniquement à au moins un élément annulaire de contact électroconducteur (4).

6. Partie de tête selon la revendication 4 ou 5, **caractérisée en ce qu'**au moins un capteur (19) peut être sélectionné à partir du groupe des capteurs suivants : capteur d'ultrasons, capteur optique, capteur de pression, capteur à effet Hall, capteur de débit.

7. Partie de tête selon l'une quelconque des revendications 4 à 6,
*caractérisée en ce qu*'au moins un capteur (19) est relié à une unité d'évaluation et de commande (22), qui est reliée à une unité de mémorisation et/ou à la source de milieu (12).

8. Partie de tête selon l'une quelconque des revendications 1 à 7,
**caractérisée en ce que** le moyen de liaison (8) comporte un profil d'assemblage (18) comprenant le conduit de milieu (11) débouchant ouvert dans la prise de contact à fiche de type à trou borgne (5), qui est structuré et disposé de telle manière qu'un deuxième conduit de milieu (17) disposé à l'intérieur de la prise de contact à fiche (5) peut être couplé au conduit de milieu (11) débouchant ouvert dans la prise de contact à fiche de type à trou borgne (5).

9. Partie de tête selon l'une quelconque des revendications 1 à 8,
**caractérisée en ce que** le boîtier de partie de tête est relié mécaniquement et électriquement à un dispositif médical (2) implantable constitué sous la forme d'un générateur d'impulsions.

10. Partie de tête selon la revendication 9,
**caractérisée en ce que** la source de milieu (12) est intégrée dans le dispositif médical implantable (2).

11. Ensemble fiche implantable constitué et adapté pour l'introduction dans la prise de contact à fiche (5) à l'intérieur de la partie de tête (1) selon l'une quelconque des revendications 1 à 10,
**caractérisé en ce que** l'ensemble fiche (13) comporte un corps de fiche (14) avec au moins un conduit creux à l'intérieur, qui débouche d'un côté sur l'extrémité de corps de fiche distale, et
**en ce que** l'extrémité de corps de fiche distale comporte un profil d'assemblage (18), qui garantit à l'état assemblé de l'ensemble fiche (13) à l'intérieur de la prise de contact à fiche (5), un assemblage affleurant du conduit creux sur le conduit de milieu (11) débouchant ouvert dans la prise de contact à fiche (5) de type à trou borgne.

12. Ensemble fiche selon la revendication 11,
**caractérisé en ce que** le conduit creux comprend un conduit de milieu (11) à la manière d'un conduit de milieu (11) débouchant ouvert dans la prise de contact à fiche (5) de type trou borgne.

13. Procédé de fabrication d'une partie de tête (1) selon l'une quelconque des revendications 1 à 10,
**caractérisé en ce que** le moyen de liaison (8), au moins un élément annulaire de contact (4) ainsi qu'au moins une bague d'étanchéité (3) sont disposés le long d'un outil de montage en forme de tige et sont assemblés par conformité de force l'un contre l'autre le long d'un outil de montage en forme de tige par génération d'une force d'assemblage,
**en ce qu'**au moins le moyen de liaison (8) disposé le long de l'outil de montage en forme de tige et assemblé par conformité de force, au moins un élément annulaire de contact (4) ainsi qu'au moins une bague d'étanchéité (3) et au moins un conduit de milieu (11) placé indirectement ou directement sur le moyen de liaison (8) sont enveloppés au moins en partie par une masse de remplissage solidifiable, présente sous une forme fluide,
**en ce que** l'outil de montage en forme de tige est libéré et enlevé du moyen de liaison (8), d'au moins un élément annulaire de contact (4) ainsi que d'au moins une bague d'étanchéité (3) après solidification de la masse de remplissage, qui soutient mécaniquement la force axiale sous la forme d'une matrice de forme stable, formant au moins une partie du boîtier de partie de tête.
